(19) **Europäisches Patentamt / European Patent Office / Office européen des brevets**

(11) **EP 4 316 602 A1**

(12) # EUROPEAN PATENT APPLICATION
published in accordance with Art. 153(4) EPC

(43) Date of publication:
**07.02.2024 Bulletin 2024/06**

(21) Application number: **22780360.8**

(22) Date of filing: **23.03.2022**

(51) International Patent Classification (IPC):
**A61Q 17/04** *(2006.01)*    **A61K 8/35** *(2006.01)*
**A61K 8/37** *(2006.01)*    **A61K 8/86** *(2006.01)*

(52) Cooperative Patent Classification (CPC):
**A61K 8/35; A61K 8/37; A61K 8/86; A61Q 17/04**

(86) International application number:
**PCT/JP2022/013395**

(87) International publication number:
**WO 2022/210145 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2021 JP 2021057854**

(71) Applicant: **Shiseido Company, Ltd.**
**Chuo-ku**
**Tokyo 104-0061 (JP)**

(72) Inventors:
- **SAKAE Heini**
  **Tokyo 104-0061 (JP)**
- **IWAMI Shiho**
  **Tokyo 104-0061 (JP)**
- **NISHIDA Yurika**
  **Tokyo 104-0061 (JP)**
- **KANG Jasmin**
  **Tokyo 104-0061 (JP)**

(74) Representative: **Ter Meer Steinmeister & Partner**
**Patentanwälte mbB**
**Nymphenburger Straße 4**
**80335 München (DE)**

(54) ## COSMETIC

(57)    Provided is a cosmetic capable of achieving improvement of an ultraviolet protection effect, particularly improvement against photodegradation.

A cosmetic is used that includes an ultraviolet absorber including t-butyl methoxydibenzoylmethane and includes butyloctyl salicylate and an alkylene oxide derivative.

EP 4 316 602 A1

**Description**

TECHNICAL FIELD

The present invention relates to a cosmetic that includes an ultraviolet absorber including t-butyl methoxydibenzoylmethane, butyloctyl salicylate and an alkylene oxide derivative.

BACKGROUND ART

Protecting the skin from damage caused by ultraviolet rays is one of important problems in skin care and body care, and various UV care cosmetics have been developed in order to minimize adverse effects of ultraviolet rays on the skin. A sunscreen cosmetic, which is a kind of UV care cosmetic, contains an ultraviolet absorber or an ultraviolet scattering agent blended and thus prevents UVA and UVB from reaching the skin to protect the skin from damage caused by ultraviolet rays. Therefore, a UV care cosmetic has been desired that has an ultraviolet protection effect even under severe ultraviolet conditions in outdoor activities such as bathing in pool or sea water in summer and skiing in winter.

There are several kinds of ultraviolet absorbers, and examples of them include ethylhexyl methoxycinnamate and octocrylene. Ethylhexyl methoxycinnamate and octocrylene are a liquid ultraviolet absorber having an absorption wavelength mainly in the UVB region, and are used in very many sunscreen cosmetics due to the excellent ultraviolet absorbability and the easy availability.

However, these ultraviolet absorbers are known to stimulate the skin of a user with sensitive skin. Regardless of the sensitive skin, in a case where applied to the face, the ultraviolet absorbers have a problem that they are moved by sebum, sweat, or the like on the skin, and enter the eye from, for example, the outer corner of the eye to give a sharp pain to the eye.

Therefore, it is desired to develop a cosmetic having an excellent ultraviolet absorbing effect by using an ultraviolet absorber substituting for ethylhexyl methoxycinnamate and octocrylene.

Patent Literature 1 discloses a sunscreen composition containing an ultraviolet absorber, diisopropyl sebacate, and at least one oil selected from ethylhexyl methoxycrylene and butyloctyl salicylate, but does not disclose any cosmetic including an alkylene oxide derivative, and does not describe at all improvement of the drawback that, in the case of blending an ultraviolet absorber having poor photostability, the ultraviolet protection ability deteriorates due to photoirradiation (hereinafter, also referred to as "photodegradation").

CITATION LIST

PATENT LITERATURE

Patent Literature 1: JP 2016-11290 A

SUMMARY OF THE INVENTION

The present inventors have surprisingly found that a cosmetic capable of achieving improvement of an ultraviolet protection effect, particularly improvement against photodegradation can be obtained by using a cosmetic that includes an ultraviolet absorber including t-butyl methoxydibenzoylmethane, butyloctyl salicylate and an alkylene oxide derivative. The present invention is based on these findings.

Therefore, the present invention discloses a cosmetic including (A) an ultraviolet absorber including t-butyl methoxydibenzoylmethane, (B) butyloctyl salicylate, and (C) an alkylene oxide derivative.

According to the present invention, the following invention is provided.

(1) A cosmetic including:

(A) an ultraviolet absorber including t-butyl methoxydibenzoylmethane;
(B) butyloctyl salicylate; and
(C) an alkylene oxide derivative.

(2) The cosmetic according to (1), wherein (C) the alkylene oxide derivative is a compound represented by Formula (I) described below:

$$R^1O\text{-}[(AO)_p(EO)_q]\text{-}R^2 \cdots \quad (I)$$

wherein

AO represents an oxyalkylene group having 3 to 4 carbon atoms, EO represents an oxyethylene group, p represents an average number of moles of added oxyalkylene groups having 3 to 4 carbon atoms and satisfies $1 \leq p \leq 70$, q represents an average number of moles of added oxyethylene groups and satisfies $1 \leq q \leq 70$, a ratio of the oxyethylene groups to a total of the oxyalkylene groups and the oxyethylene groups is 20 to 80 mass%, the oxyalkylene groups and the oxyethylene groups are added in a block form or a random form,
$R^1$ and $R^2$ are identical or different and each represent a hydrocarbon group having 1 to 4 carbon atoms or a hydrogen atom, and a ratio of a number of hydrogen atoms represented by $R^1$ and $R^2$ to a number of hydrocarbon groups represented by $R^1$ and $R^2$ is 0.15 or less.

(3) The cosmetic according to (2), wherein in (C) the alkylene oxide derivative, the oxyalkylene groups and the oxyethylene groups are added in a random form.
(4) The cosmetic according to any one of (1) to (3), wherein an amount of ethylhexyl methoxycinnamate blended in the cosmetic is 2 mass% or less with respect to a total amount of the cosmetic.
(5) The cosmetic according to any one of (1) to (4), wherein an amount of octocrylene blended in the cosmetic is 2 mass% or less with respect to the total amount of the cosmetic.
(6) The cosmetic according to any one of (1) to (5), being an oily cosmetic.
(7) The cosmetic according to any one of (1) to (6), being a water-in-oil emulsion cosmetic.
(8) The cosmetic according to any one of (1) to (7), being a sunscreen cosmetic.

Using the cosmetic of the present invention is advantageous in that a cosmetic can be obtained that is capable of achieving improvement of an ultraviolet protection effect, particularly improvement against photodegradation.

BEST MODE FOR CARRYING OUT THE INVENTION

The present invention is
a cosmetic including:

(A) an ultraviolet absorber including t-butyl methoxydibenzoylmethane;
(B) butyloctyl salicylate; and
(C) an alkylene oxide derivative.

(A) Ultraviolet Absorber

The cosmetic of the present invention includes (A) an ultraviolet absorber including t-butyl methoxydibenzoylmethane. As the ultraviolet absorber included in the cosmetic of the present invention, an ultraviolet absorber usually used in sunscreen cosmetics can be used in addition to t-butyl methoxydibenzoylmethane.

The ultraviolet absorber usable in addition to t-butyl methoxydibenzoylmethane is not particularly limited, and is exemplified by a wide range of ultraviolet absorbers used in cosmetics generally. Specific examples of the ultraviolet absorbers include benzoic acid derivatives, salicylic acid derivatives, cinnamic acid derivatives, dibenzoylmethane derivatives, β,β-diphenylacrylate derivatives, benzophenone derivatives, benzylidene camphor derivatives, phenylbenzimidazole derivatives, triazine derivatives, phenylbenzotriazole derivatives, anthranil derivatives, imidazoline derivatives, benzalmalonate derivatives, and 4,4-diarylbutadiene derivatives.

Examples of the benzoic acid derivatives include ethyl para-aminobenzoate (PABA), ethyl-dihydroxypropyl PABA, ethylhexyl-dimethyl PABA, glyceryl PABA, PEG-25-PABA, and hexyl diethylamino hydroxybenzoyl benzoate.

Examples of the salicylic acid derivatives include homosalate, ethylhexyl salicylate, dipropylene glycol salicylate, and TEA salicylate.

Examples of the cinnamic acid derivatives include octylmethoxy cinnamate, isopropyl methoxycinnamate, isoamyl methoxycinnamate, cinnoxate, DEA methoxycinnamate, diisopropyl methyl cinnamate, glyceryl-ethylhexanoate-dimethoxycinnamate, and di-(2-ethylhexyl)-4'-methoxybenzalmalonate.

Examples of the dibenzoylmethane derivatives include 5-(3,3-dimethyl-2-norbornylidene) -3 -pentan-2-one.

Examples of the β,β-diphenylacrylate derivatives include etocrylene.

Examples of the benzophenone derivatives include benzophenone-1, benzophenone-2, benzophenone-3 or oxybenzone, benzophenone-4, benzophenone-5, benzophenone-6, benzophenone-8, benzophenone-9, and benzophenone-12.

Examples of the benzylidene camphor derivatives include 3-benzylidene camphor, 4-methylbenzylidene camphor, benzylidene camphor sulfonic acid, camphor benzalkonium methosulfate, terephthalylidene dicamphor sulfonic acid, and polyacrylamidomethyl benzylidene camphor.

Examples of the phenylbenzimidazole derivatives include phenylbenzimidazole sulfonic acid and disodium phenyl dibenzimidazole tetrasulfonate.

Examples of the triazine derivatives include bisethylhexyloxyphenol methoxyphenyltriazine, ethylhexyl triazone, diethylhexyl butamido triazone, 2,4,6-tris(diisobutyl-4'-aminobenzalmalonate)-s-triazine, and 2,4,6-tris[4-(2-ethylhexyloxycarbonyl)anilino]-1,3,5-triazine.

Examples of the phenylbenzotriazole derivatives include drometrizole trisiloxane and methylenebis(benzotriazolyltetramethylbutylphenol).

Examples of the anthranil derivatives include menthyl anthranilate.

Examples of the imidazoline derivatives include ethylhexyl dimethoxybenzylidene dioxoimidazoline propionate.

Examples of the benzalmalonate derivatives include polyorganosiloxanes having a benzalmalonate functional group.

Examples of the 4,4-diarylbutadiene derivatives include 1,1-dicarboxy(2,2'-dimethylpropyl)-4,4-diphenylbutadiene.

The amount of (A) the ultraviolet absorber blended in the cosmetic according to the present invention is not particularly limited, and is 5 to 40 mass%, more preferably 6 to 40 mass%, and still more preferably 7 to 30 mass% with respect to the total amount of the cosmetic. (A) The ultraviolet absorber may be used singly or in combination of two or more kinds thereof.

The amount of t-butyl methoxydibenzoylmethane blended in (A) the ultraviolet absorber blended in the cosmetic according to the present invention is not particularly limited, and is 0.5 to 5 mass%, more preferably 0.5 to 4 mass%, and still more preferably 1 to 2.5 mass% with respect to the total amount of the cosmetic.

According to a preferred aspect of the cosmetic of the present invention, the amount of ethylhexyl methoxycinnamate blended in the cosmetic of the present invention is 2 mass% or less, more preferably 1 mass% or less, and still more preferably 0.5 mass% or less with respect to the total amount of the cosmetic. The cosmetic of the present invention also includes an aspect in which ethylhexyl methoxycinnamate is not included or substantially not included.

According to a preferred aspect of the cosmetic of the present invention, the amount of octocrylene blended in the cosmetic of the present invention is 2 mass% or less, more preferably 1 mass% or less, and still more preferably 0.5 mass% or less with respect to the total amount of the cosmetic. The cosmetic of the present invention also includes an aspect in which octocrylene is not included or substantially not included.

According to a preferred aspect of the cosmetic of the present invention, the total amount of ethylhexyl methoxycinnamate and octocrylene blended in the cosmetic of the present invention is 3 mass% or less, more preferably 2 mass% or less, and still more preferably 1 mass% or less with respect to the total amount of the cosmetic.

(B) Butyloctyl Salicylate

The cosmetic of the present invention includes (B) butyloctyl salicylate. As the butyloctyl salicylate used in the cosmetic of the present invention, a commercially available product such as HALLBRITE BHB (trade name, manufactured by Hall Star) can be used.

The amount of (B) butyloctyl salicylate blended in the cosmetic of the present invention is not particularly limited, and is preferably 1 to 10 mass% with respect to the total amount of the cosmetic, and more preferably 2 to 8 mass% with respect to the total amount of the cosmetic. If the amount of butyloctyl salicylate blended in the cosmetic of the present invention is set to 2 to 8 mass% with respect to the total amount of the cosmetic, further improvement of the ultraviolet protection effect, particularly further improvement against photodegradation can be achieved.

(C) Alkylene Oxide Derivative

The cosmetic of the present invention includes (C) an alkylene oxide derivative. (C) The alkylene oxide derivative included in the cosmetic of the present invention is not particularly limited, and is preferably a compound represented by Formula (I) described below.

$$R^1O\text{-}[(AO)_p(EO)_q]\text{-}R^2 \cdots \quad (I)$$

In the formula,

AO represents an oxyalkylene group having 3 to 4 carbon atoms, EO represents an oxyethylene group, p represents an average number of moles of added oxyalkylene groups having 3 to 4 carbon atoms and satisfies $1 \leq p \leq 70$, q represents an average number of moles of added oxyethylene groups and satisfies $1 \leq q \leq 70$, a ratio of the oxyethylene groups to a total of the oxyalkylene groups and the oxyethylene groups is 20 to 80 mass%,

the oxyalkylene groups and the oxyethylene groups are added in a block form or a random form,

$R^1$ and $R^2$ are identical or different and each represent a hydrocarbon group having 1 to 4 carbon atoms or a hydrogen atom, and a ratio of a number of hydrogen atoms represented by $R^1$ and $R^2$ to a number of hydrocarbon groups represented by $R^1$ and $R^2$ is 0.15 or less.

In the compound represented by Formula (I) described above, AO represents an oxyalkylene group having 3 to 4 carbon atoms, and examples of the oxyalkylene group include an oxypropylene group, an oxybutylene group, an oxyisobutylene group, a trimethylene group, and a tetramethylene group, and an oxypropylene group or an oxybutylene group is preferable. p represents an average number of moles of added oxyalkylene groups having 3 to 4 carbon atoms, and satisfies $1 \leq p \leq 70$ and preferably satisfies $2 \leq p \leq 20$. q represents an average number of moles of added oxyethylene groups, and satisfies $1 \leq q \leq 70$ and preferably satisfies $2 \leq q \leq 20$. Furthermore, (p + q) is preferably 8 to 100.

In the compound represented by Formula (I) described above, the ratio of the oxyethylene groups to the total of the oxyalkylene groups having 3 to 4 carbon atoms and the oxyethylene groups is preferably 20 to 80 mass%. The order of addition of ethylene oxide and an alkylene oxide having 3 to 4 carbon atoms is not particularly limited. The oxyethylene groups and the oxyalkylene groups having 3 to 4 carbon atoms may be added in a block form or a random form, and are preferably added in a random form. Here, the block form includes not only a two-stage block but also a three-stage block.

$R^1$ and $R^2$ used may be the same, a combination of a hydrocarbon group having 1 to 4 carbon atoms and a hydrogen atom, or a combination of different hydrocarbon groups having 1 to 4 carbon atoms. However, in the hydrocarbon groups represented by $R^1$ and $R^2$, the existence ratio between the hydrocarbon groups and the hydrogen atoms is such that the ratio Y/X of the number of the hydrogen atoms (Y) to the number of the hydrocarbon groups (X) is 0.15 or less, and preferably 0.06 or less.

Specific examples of the alkylene oxide derivative include polyoxyethylene (10 mol) polyoxypropylene (10 mol) dimethyl ether, polyoxyethylene (9 mol) polyoxypropylene (2 mol) dimethyl ether, polyoxyethylene (14 mol) polyoxypropylene (7 mol) dimethyl ether, polyoxyethylene (6 mol) polyoxypropylene (14 mol) dimethyl ether, polyoxyethylene (15 mol) polyoxypropylene (5 mol) dimethyl ether, polyoxyethylene (25 mol) polyoxypropylene (25 mol) dimethyl ether, polyoxyethylene (9 mol) polyoxybutylene (2 mol) dimethyl ether, polyoxyethylene (14 mol) polyoxybutylene (7 mol) dimethyl ether, polyoxyethylene (10 mol) polyoxypropylene (10 mol) diethyl ether, polyoxyethylene (10 mol) polyoxypropylene (10 mol) dipropyl ether, polyoxyethylene (10 mol) polyoxypropylene (10 mol) dibutyl ether, and polyoxyethylene (36 mol) polyoxypropylene (41 mol) dimethyl ether, and among them, polyoxyethylene (9 mol) polyoxypropylene (2 mol) dimethyl ether, polyoxyethylene (14 mol) polyoxypropylene (7 mol) dimethyl ether, or polyoxyethylene (36 mol) polyoxypropylene (41 mol) dimethyl ether is preferable, and polyoxyethylene (9 mol) polyoxypropylene (2 mol) dimethyl ether is particularly preferable.

The alkylene oxide derivative of the present invention can be produced with a known method. For example, the alkylene oxide derivative of the present invention can be obtained by addition-polymerizing ethylene oxide and an alkylene oxide having 3 to 4 carbon atoms to a compound having a hydroxy group, and then etherifying an alkyl halide in the presence of an alkali catalyst.

The amount of (C) the alkylene oxide derivative blended in the cosmetic of the present invention is not particularly limited, and is preferably 1 to 15 mass% with respect to the total amount of the cosmetic, and more preferably 8 to 12 mass% with respect to the total amount of the cosmetic. If the amount of the alkylene oxide derivative blended is set to 1 to 15 mass% with respect to the total amount of the cosmetic, further improvement of the ultraviolet protection effect, particularly further improvement against photodegradation can be achieved.

## (D) Ester Oil

The cosmetic of the present invention preferably includes (D) an ester oil from the viewpoint of improvement against photodegradation. In the present invention, (D) the ester oil means an ester oil other than butyloctyl salicylate and an ultraviolet absorber. (D) The ester oil included in the cosmetic of the present invention is not particularly limited, and examples of the ester oil include diethylhexyl succinate, an alkyl benzoate (C12 to 15), phenethyl benzoate, diisopropyl sebacate, cetyl 2-ethylhexanoate, glyceryl tri-2-ethylhexanoate, dibutyl adipate, 2-ethylhexyl palmitate, isopropyl palmitate, isopropyl myristate, isopropyl stearate, isobutyl stearate, 2-ethylhexyl stearate, isopropyl isostearate, butyl isostearate, decyl isostearate, lauryl isostearate, isodecyl isodecanoate, isodecyl isononanoate, isotridecyl isononanoate, isononyl isononanoate, neopentyl glycol dioctanoate, glyceryl tri-2-ethylhexanoate, propylene glycol dicaprate, propylene glycol dicaprate, caprylic/capric triglyceride, trimethylolpropane triisostearate, pentaerythritol tetra-2-ethylhexanoate, and dipentaerythrite fatty acid ester, and among these, diethylhexyl succinate, an alkyl benzoate (C12 to 15), or diethylhexyl sebacate is preferably included, and a combination of diethylhexyl succinate and an alkyl benzoate (C12 to 15) is more preferably included.

The amount of (D) the ester oil blended in the cosmetic of the present invention is not particularly limited, and is preferably 5 to 30 mass% with respect to the total amount of the cosmetic, and more preferably 5 to 25 mass% with respect to the total amount of the cosmetic.

In a case where (D) the ester oil included in the cosmetic of the present invention contains diethylhexyl succinate and/or an alkyl benzoate (C12 to 15), the amount of the diethylhexyl succinate and/or the alkyl benzoate (C12 to 15) blended (in the case of containing both of them, the sum of the amounts of them) is preferably 5 to 10 mass% with respect to the total amount of the cosmetic. Thus, further improvement of the ultraviolet protection effect, particularly further improvement against photodegradation can be achieved.

The cosmetic of the present invention may include, in addition to the above components, another optional component such as an inorganic powder, an organic powder, a water-soluble polymer, an oil-soluble polymer, a wax, an alcohol, a hydrocarbon oil, a fatty acid, a higher alcohol, a fatty acid ester, a silicone oil, a surfactant, or a drug.

The inorganic powder that may be included in the cosmetic of the present invention is not particularly limited as long as it is a powder used in the field of cosmetics. Specific examples include one or more selected from titanium oxide, zinc oxide, cerium oxide, barium sulfate, iron oxide, talc, mica, sericite, kaolin, mica titanium, Prussian blue, chromium oxide, chromium hydroxide, silica, and the like.

As an ultraviolet scattering agent, a powder having a refractive index of 1.5 or more, such as zinc oxide, titanium oxide, or cerium oxide, is particularly preferably used from the viewpoint of optical characteristics.

Surface hydrophobization treatment of an ultraviolet scattering agent improves the dispersibility in oil and the water resistance, and therefore a surface-hydrophobized ultraviolet scattering agent may be preferably included. Examples of the surface treatment method include silicone treatment with methylhydrogenpolysiloxane, methylpolysiloxane, or the like, alkylsilane treatment, fluorine treatment with a perfluoroalkyl phosphate ester, a perfluoroalcohol, or the like, amino acid treatment with an N-acylglutamic acid or the like, lecithin treatment, metal soap treatment, fatty acid treatment, and alkyl phosphate ester treatment.

The ultraviolet scattering agent that may be included in the cosmetic of the present invention is not particularly limited, and usually preferably has an average primary particle size of 100 nm or less and more preferably 80 nm or less. In a case where the average primary particle size is much more than 100 nm, white smear or white residue tends to be caused. The average primary particle size in the present invention is, for example, a value determined as an arithmetic mean of the long diameter and the short diameter of a particle from a transmission electron micrograph.

The particle shape of the ultraviolet scattering agent is not particularly limited, and the ultraviolet scattering agent may be in a state of primary particles or may be formed into an aggregated secondary aggregate. Furthermore, the shape such as a spherical shape, an elliptical shape, a crushed shape, or the like is not particularly limited.

Examples of the water-soluble polymer that may be included in the cosmetic of the present invention include a homopolymer and copolymers of 2-acrylamido-2-methylpropanesulfonic acid (hereinafter, abbreviated as "AMPS"). The copolymers are a copolymer including a comonomer such as vinylpyrrolidone, acrylic acid amide, sodium acrylate, or hydroxyethyl acrylate. That is, examples thereof include an AMPS homopolymer, a vinylpyrrolidone/AMPS copolymer, a dimethylacrylamide/AMPS copolymer, an acrylic acid amide/AMPS copolymer, and a sodium acrylate/AMPS copolymer.

Furthermore, examples thereof include a carboxyvinyl polymer, ammonium polyacrylate, sodium polyacrylate, a sodium acrylate/alkyl acrylate/sodium methacrylate/alkyl methacrylate copolymer, carrageenan, pectin, mannan, curdlan, chondroitin sulfate, starch, glycogen, gum arabic, sodium hyaluronate, tragacanth gum, xanthan gum, mucoitinsulfuric acid, hydroxyethyl guar gum, carboxymethyl guar gum, guar gum, dextran, keratosulfate, locust bean gum, succinoglycan, chitin, chitosan, carboxymethyl chitin, and agar.

Examples of the oil-soluble polymer include trimethylsiloxysilicate, alkyl-modified silicone, polyamide-modified silicone, a dimethicone crosspolymer, a (dimethicone/vinyl dimethicone) crosspolymer, and polymethylsilsesquioxane.

Examples of the wax include beeswax, candelilla wax, carnauba wax, and jojoba wax.

Examples of the alcohol include lower alcohols such as ethanol and isopropanol, and polyhydric alcohols such as ethylene glycol, propylene glycol, 1,3-butylene glycol, dipropylene glycol, and polybutylene glycol.

Examples of the hydrocarbon oil include liquid paraffin, ozokerite, squalane, pristane, paraffin, ceresin, squalene, petrolatum, microcrystalline wax, polyethylene wax, and Fischer-Tropsch wax.

Examples of the fatty acid include lauric acid, myristic acid, palmitic acid, stearic acid, behenic acid, and arachidonic acid.

Examples of the higher alcohol include lauryl alcohol, myristyl alcohol, cetyl alcohol, stearyl alcohol, behenyl alcohol, aralkyl alcohol, batyl alcohol, chimyl alcohol, carnaubyl alcohol, ceryl alcohol, corianyl alcohol, myricyl alcohol, lacceryl alcohol, elaidyl alcohol, isostearyl glyceryl ether, octyl alcohol, triacontyl alcohol, selachyl alcohol, cetostearyl alcohol, oleyl alcohol, lanolin alcohol, hydrogenated lanolin alcohol, isostearyl alcohol, hexyldecanol, and octyldecanol.

Examples of the silicone oil include methylpolysiloxane, octamethylsiloxane, decamethyltetrasiloxane, methylhydrogenpolysiloxane, methylphenylpolysiloxane, hexamethylcyclotrisiloxane, octamethylcyclotetrasiloxane, and decamethylcyclopentasiloxane. Preferred examples of the silicone oil include octamethylcyclotetrasiloxane and decamethylcyclopentasiloxane.

Examples of the surfactant include anionic, cationic, nonionic, and amphoteric surfactants, including silicone-based and hydrocarbon-based surfactants.

Examples of the powder component other than the ultraviolet scattering agent include nylon-based and acrylic-based polymer spherical powders, silica powders, silicone powders, and metal oxide powders surface-treated with a metal-free surface treatment agent.

Examples of the drug include salts of L-ascorbic acid and its derivatives, glycyrrhizic acid and its derivatives such as dipotassium glycyrrhizinate and monoammonium glycyrrhizinate, glycyrrhetinic acid and its derivatives such as stearyl glycyrrhetinate, allantoin, salts of tranexamic acid and its derivatives, salts of alkoxysalicylic acid and its derivatives, salts of glutathione and its derivatives, allantoin, and azulene.

According to one preferred aspect of the cosmetic of the present invention, the cosmetic is an oily cosmetic. Here, the term "oily cosmetic" means a one-oily-phase (anhydrous) or water-in-oil cosmetic.

According to a further preferred aspect of the cosmetic of the present invention, the cosmetic is a water-in-oil emulsion cosmetic.

According to another preferred aspect of the cosmetic of the present invention, the cosmetic is a sunscreen cosmetic, and the sunscreen cosmetic may be an oily cosmetic or a water-in-oil emulsion cosmetic. Specific forms include forms of a sunscreen emulsion and a sunscreen cream, which can be produced using an ordinary method suitable for respective forms.

[Examples]

The present invention will be described specifically with reference to the following Examples, but the present invention is not limited to these Examples. The amount of a blended component is shown in mass% unless otherwise specified.

Cosmetics having compositions shown in Tables 1 and 2 below (Examples 1 to 6 and Comparative Examples 1 to 6) were prepared with an ordinary method, and the residual rates (%) were calculated in accordance with the following method. Tables 1 and 2 describe the calculated residual rates (%).

(1) Calculation of Residual Rate

For each cosmetic (Examples 1 to 6 and Comparative Examples 1 to 6), the photostability (stability of ultraviolet absorbability) of the sunscreen cosmetic was evaluated with the following method. Each sample was applied to a PMMA substrate, and the absorbance at 500 to 280 nm was measured. The substrate was irradiated with UV in an amount at an erythema UV intensity of 16 MED (minimal erythema dose)/min/cm$^2$ using an SPF measuring device "SPF MASTER" (registered trademark) (Shiseido Company, Limited), and then the absorbance was measured in the same manner, and a change in the integrated value of absorbance in the above-described wavelength region between before and after the irradiation (residual rate of ultraviolet absorbability after photoirradiation) (in vitro) was calculated in accordance with the following formula.

$$\text{Residual rate (\%) after photoirradiation} = (\text{integrated value of absorbance after photoirradiation})/(\text{integrated value of absorbance before photoirradiation}) \times 100$$

Evaluation of the residual rate in accordance with the following evaluation criteria is shown as "A" to "D" in Tables 1 and 2 below.

<Evaluation Criteria>

A: Residual rate is 90% or more
B: Residual rate is 85% or more and less than 90%
C: Residual rate is 80% or more and less than 85%
D: Residual rate is less than 80%

*The numerical values in the table are shown in mass%.

Table 1: Examples 1 to 6

| Component name | Example 1 | Example 2 | Example 3 | Example 4 | Example 5 | Example 6 |
|---|---|---|---|---|---|---|
| Ion exchanged water | Balance | Balance | Balance | Balance | Balance | Balance |
| Denatured alcohol | 4 | 4 | 4 | 4 | 4 | 4 |
| Polyoxyethylene (9) polyoxypropylene (2) dimethyl ether | 10 | 10 | 10 | 10 | 10 | 10 |
| Glycerine | 2 | 2 | 2 | 2 | 2 | 2 |
| Dimethyldistearyl ammonium hectorite | 1 | 1 | 0.7 | | 0. 7 | 0. 7 |
| Dextrin palmitate | 2 | 2 | 2 | 2 | 2 | 2 |
| Sucrose tetrastearate triacetate | 2 | 2 | 2 | 2 | 2 | 2 |
| Lauryl PEG-9 polydimethylpolysiloxyethyl dimethicone | 4 | 4 | 3.2 | 3.2 | 3.2 | 3. 2 |
| Isododecane | 4 | 4 | 4. 5 | 4. 5 | 4. 5 | 4. 5 |
| Dimethicone (1.5 cst) | - | - | - | - | - | - |
| Isostearic acid | 0.5 | 0.5 | 0. 45 | 0. 45 | 0. 45 | 0. 45 |
| Triethylhexanoin | 3 | 3 | 3 | 3 | 3 | 2 |
| Diisopropyl sebacate | 10 | 10 | 10 | - | - | 10 |
| PPG-3 dipivalate | 2 | 2 | 2 | 4. 3 | 4.3 | 2 |
| Diethylhexyl succinate | - | - | - | 4 | 4 | - |
| Butyloctyl salicylate | 5 | 5 | 5 | 5 | 5 | 5 |
| Phenethyl benzoate | - | - | - | - | - | - |
| Alkyl benzoate (C12 to 15) | - | - | - | 3. 7 | 3. 7 | - |
| Adipic acid dibutyl ester | - | - | - | - | - | - |
| Trimethylsiloxysilicate methyl polysiloxane | 3 | 3 | 3 | 3 | 3 | 3 |
| Butyl methoxydibenzoylmethane | 2 | 2.3 | 2 | 2 | 2 | 2. 3 |
| Ethylhexyl salicylate | 5 | 5 | 5 | - | - | 5 |
| Homosalate | 15 | 15 | 15 | - | - | 15 |
| Bis-ethylhexyloxyphenol methoxyphenyl triazine | - | - | - | 1. 5 | 1. 5 | - |
| Polvsilicone-15 | - | - | - | 3 | 3 | - |
| Ethylhexyl triazone | - | - | - | - | 1. 5 | - |
| Phenylbenzimidazole sulfonic acid | - | - | - | - | 2 | - |
| Talc | - | - | 10 | 10 | 10 | - |
| Silica | 1 | 1 | - | - | - | 1 |
| Silica dimethyl silylate | - | - | - | - | - | 0. 1 |
| Dibutylhydroxytoluene | - | - | 0.05 | 0. 05 | 0. 05 | 0. 05 |

(continued)

| Component name | Example 1 | Example 2 | Example 3 | Example 4 | Example 5 | Example 6 |
|---|---|---|---|---|---|---|
| Sodium metabisulfite | - | - | 0. 01 | 0. 01 | 0. 01 | 0. 01 |
| Tocopherol | - | - | 0. 05 | 0. 05 | 0. 05 | 0. 05 |
| Sodium chloride | - | - | 0. 1 | 0. 1 | 0. 1 | 0. 1 |
| EDTA-3Na. $2H_2O$ | 0. 1 | 0. 1 | 0. 1 | 0. 1 | 0. 1 | 0. 1 |
| Methylparaben | 0. 15 | 0. 15 | 0. 15 | 0. 15 | 0. 15 | 0. 15 |
| Total | 100 | 100 | 100 | 100 | 100 | 100 |
| Residual rate (%) | 92. 77 | 88.23 | 87. 13 | 92. 59 | 97. 49 | 85. 3 |
| Evaluation of residual rate | A | B | B | A | A | B |

*The numerical values in the table are shown in mass%.

Table 2: Comparative Examples 1 to 6

| Component name | Comparative Example 1 | Comparative Example 2 | Comparative Example 3 | Comparative Example 4 | Comparative Example 5 | Comparative Example 6 |
|---|---|---|---|---|---|---|
| Ion exchanged water | Balance | Balance | Balance | Balance | Balance | Balance |
| Denatured alcohol | 4 | 4 | 4 | 4 | 4 | 4 |
| Polyoxyethylene (9) polyoxypropylene (2) dimethyl ether | 10 | - | 10 | 10 | 10 | - |
| Glycerine | 2 | 2 | 2 | 2 | 2 | 2 |
| Dimethyldistearyl ammonium hectorite | 0.7 | 0.7 | 0.7 | 0.7 | 0.7 | 0.7 |
| Dextrin palmitate | 2 | 2 | 2 | 2 | 2 | 2 |
| Sucrose tetrastearate triacetate | 2 | 2 | 2 | 2 | 2 | 2 |
| Lauryl PEG-9 polydimethylpolysiloxyethyl dimethicone | 4 | 4 | 3.2 | 3.2 | 3.2 | 3.2 |
| Isododecane | 4 | 4 | 4.5 | 4.5 | 4.5 | 4.5 |
| Dimethicone (1.5 cst) | 4 | - | - | - | - | 4 |
| Isostearic acid | 0.45 | 0.45 | 0.45 | 0.45 | 0.45 | 0.45 |
| Triethylhexanoin | 2 | 3 | 3 | 3 | 3 | 2 |
| Diisopropyl sebacate | 10 | 10 | 10 | 10 | 10 | 10 |
| PPG-3 dipivalate | 2 | 2 | 2 | 2 | 2 | 2 |
| Diethylhexyl succinate | - | - | - | - | - | - |
| Butyloctyl salicylate | - | 5 | - | - | - | - |
| Phenethyl benzoate | - | - | 5 | - | - | - |
| Alkyl benzoate (C12 to 15) | - | - | - | 5 | - | - |
| Adipic acid dibutyl ester | - | - | - | - | 5 | - |
| Trimethylsiloxysilicate methyl polysiloxane | 3 | 3 | 3 | 3 | 3 | 3 |
| Butyl methoxydibenzoylmethane | 2.3 | 2.3 | 2 | 2 | 2 | 2.3 |
| Ethylhexyl salicylate | 5 | 5 | 5 | 5 | 5 | 5 |

(continued)

| Component name | Comparative Example 1 | Comparative Example 2 | Comparative Example 3 | Comparative Example 4 | Comparative Example 5 | Comparative Example 6 |
|---|---|---|---|---|---|---|
| Homosalate | 15 | 15 | 15 | 15 | 15 | 10 |
| Bis-ethylhexyloxyphenol methoxyphenyl triazine | - | - | - | - | - | - |
| Polvsilicone-15 | - | - | - | - | - | - |
| Ethylhexyl triazone | - | - | - | - | - | - |
| Phenylbenzimidazole sulfonic acid | - | - | - | - | - | - |
| Talc | - | - | - | - | - | - |
| Silica | 1 | 1 | 1 | 1 | 1 | 1 |
| Silica dimethyl silylate | 0.1 | 0. 1 | 0.1 | 0.1 | 0.1 | 0. 1 |
| Dibutylhydroxytoluene | 0. 05 | 0. 05 | 0. 05 | 0. 05 | 0. 05 | 0. 05 |
| Sodium metabisulfite | 0.01 | 0.01 | 0.01 | 0.01 | 0.01 | 0.01 |
| Tocopherol | 0. 05 | 0. 05 | 0. 05 | 0. 05 | 0. 05 | 0. 05 |
| Sodium chloride | 0. 1 | 0.1 | 0.1 | 0.1 | 0.1 | 0. 1 |
| EDTA-3Na. 2H$_2$0 | 0.1 | 0.1 | 0.1 | 0.1 | 0.1 | 0.1 |
| Methylparaben | 0. 15 | 0.15 | 0. 15 | 0.15 | 0.15 | 0.15 |
| Total | 100 | 100 | 100 | 100 | 100 | 100 |
| Residual rate (%) | 83.82 | 75.55 | 79.8 | 83.47 | 81. 8 | 63. 18 |
| Evaluation of residual rate | C | D | D | C | C | D |

From the results in Tables 1 and 2, in the cosmetics included in the scope of the present invention (Examples 1 to 6), the evaluation result of the residual rate was "A" or "B", and thus a high residual rate was exhibited. Meanwhile, in the cosmetics not included in the scope of the present invention (Comparative Examples 1 to 6), the evaluation result of the residual rate was "C" or "D", and thus a low residual rate was exhibited. Therefore, it has been found that the cosmetic of the present invention can achieve improvement of an ultraviolet protection effect, particularly improvement against photodegradation.

Formulation Example 1: Sunscreen

An example of a sunscreen included in the scope of the cosmetic of the present invention is shown below as Formulation Example 1.

Table 3: Formulation Example 1

| Component | Formulation Example 1 |
|---|---|
| Ion exchanged water | Balance |
| Denatured alcohol | 4 |
| Polyoxyethylene (9) polyoxypropylene (2) dimethyl ether | 10 |
| Glycerine | 2 |
| Dimethydistearyl ammonium hectorite | 1 |
| Dextrin palmitate | 2 |
| Sucrose tetrastearate triacetate | 2 |
| Lauryl PEG-9 polydimethylpolysiloxyethyl dimethicone | 4 |
| Isododecane | 4 |
| Isostearic acid | 0.5 |
| Triethylhexanoin | 3 |
| Diisopropyl sebacate | 10 |
| PPG-3 dipivalate | 2 |
| Butyloctyl salicylate | 5 |
| Trimethvlsiloxvsilicate methyl polysiloxane | 3 |
| Butyl methoxydibenzoylmethane | 2.3 |
| Ethyhexyl salicylate | 5 |
| Homosalate | 15 |
| Silica | 1 |
| Polvhvdroxvbutyrate | 0.1 |
| Polv(3-hvdroxybutyric acid-co-3-hydroxyvaleric acid) | 0.1 |
| EDTA-3Na.2H$_2$0 | 0.1 |
| Methylparaben | 0.15 |
| Total | 100 |

**Claims**

1. A cosmetic comprising:

    (A) an ultraviolet absorber including t-butyl methoxydibenzoylmethane;
    (B) butyloctyl salicylate; and

(C) an alkylene oxide derivative.

2. The cosmetic according to claim 1, wherein (C) the alkylene oxide derivative is a compound represented by Formula (I) described below:

$$R^1O\text{-}[(AO)_p(EO)_q]\text{-}R^2 \cdots \qquad (I)$$

wherein

AO represents an oxyalkylene group having 3 to 4 carbon atoms, EO represents an oxyethylene group, p represents an average number of moles of added oxyalkylene groups having 3 to 4 carbon atoms and satisfies $1 \le p \le 70$, q represents an average number of moles of added oxyethylene groups and satisfies $1 \le q \le 70$, a ratio of the oxyethylene groups to a total of the oxyalkylene groups and the oxyethylene groups is 20 to 80 mass%, the oxyalkylene groups and the oxyethylene groups are added in a block form or a random form, $R^1$ and $R^2$ are identical or different and each represent a hydrocarbon group having 1 to 4 carbon atoms or a hydrogen atom, and a ratio of a number of hydrogen atoms represented by $R^1$ and $R^2$ to a number of hydrocarbon groups represented by $R^1$ and $R^2$ is 0.15 or less.

3. The cosmetic according to claim 2, wherein in (C) the alkylene oxide derivative, the oxyalkylene groups and the oxyethylene groups are added in a random form.

4. The cosmetic according to any one of claims 1 to 3, wherein an amount of ethylhexyl methoxycinnamate blended in the cosmetic is 2 mass% or less with respect to a total amount of the cosmetic.

5. The cosmetic according to any one of claims 1 to 4, wherein an amount of octocrylene blended in the cosmetic is 2 mass% or less with respect to the total amount of the cosmetic.

6. The cosmetic according to any one of claims 1 to 5, being an oily cosmetic.

7. The cosmetic according to any one of claims 1 to 6, being a water-in-oil emulsion cosmetic.

8. The cosmetic according to any one of claims 1 to 7, being a sunscreen cosmetic.

## INTERNATIONAL SEARCH REPORT

International application No.

**PCT/JP2022/013395**

### A. CLASSIFICATION OF SUBJECT MATTER

*A61Q 17/04*(2006.01)i; *A61K 8/35*(2006.01)i; *A61K 8/37*(2006.01)i; *A61K 8/86*(2006.01)i
FI: A61K8/86; A61Q17/04; A61K8/37; A61K8/35

According to International Patent Classification (IPC) or to both national classification and IPC

### B. FIELDS SEARCHED

Minimum documentation searched (classification system followed by classification symbols)

A61Q17/04; A61K8/35; A61K8/37; A61K8/86

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2004-83541 A (SHISEIDO CO., LTD.) 18 March 2004 (2004-03-18)<br>claims 1-3, paragraphs [0014], [0032], [0044], blending examples 6-10 | 1-8 |
| Y | JP 2016-11290 A (TAISHO PHARMACEUTICAL CO., LTD.) 21 January 2016 (2016-01-21)<br>claims 1-2, paragraphs [0001], [0004], [0022], [0026], [0042], examples 7, 17, 23 | 1-8 |
| A | JP 2004-292343 A (SHISEIDO CO., LTD.) 21 October 2004 (2004-10-21)<br>claims 1-2 | 1-8 |
| A | JP 2008-88127 A (SHISEIDO CO., LTD.) 17 April 2008 (2008-04-17)<br>claim 1 | 1-8 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **17 May 2022** | **31 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/013395**

| Patent document cited in search report | | | Publication date (day/month/year) | Patent family member(s) | | | Publication date (day/month/year) |
|---|---|---|---|---|---|---|---|
| JP | 2004-83541 | A | 18 March 2004 | US claims 1-3 EP KR CN | 2010/0209370 1283031 10-2003-0027644 1408339 | A1 A2 A A | |
| JP | 2016-11290 | A | 21 January 2016 | (Family: none) | | | |
| JP | 2004-292343 | A | 21 October 2004 | (Family: none) | | | |
| JP | 2008-88127 | A | 17 April 2008 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016011290 A